(19) Europäisches Patentamt / European Patent Office / Office européen des brevets

(11) **EP 2 915 485 B1**

(12) **EUROPEAN PATENT SPECIFICATION**

(45) Date of publication and mention
of the grant of the patent:
**02.08.2017 Bulletin 2017/31**

(51) Int Cl.:
***A61B 5/16*** *(2006.01)*  ***G09B 11/04*** *(2006.01)*
***A61B 5/103*** *(2006.01)*  ***G06T 11/20*** *(2006.01)*

(21) Application number: **14000802.0**

(22) Date of filing: **06.03.2014**

(54) **Computer-implemented method and system for testing or training a user's cognitive functions**

Computerimplementiertes Verfahren und System zum Testen oder Trainieren der kognitiven Fähigkeiten eines Benutzers

Procédé et système informatisés permettant de tester ou d'entraîner les fonctions cognitives d'un utilisateur

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**09.09.2015 Bulletin 2015/37**

(73) Proprietor: **Rath, Matthias**
**Aptos, CA 95003 (US)**

(72) Inventors:
• **Rath, Matthias**
**Aptos, CA 95003 (US)**

• **Karnath, Dirk Fried**
**40219 Düsseldorf (DE)**

(74) Representative: **Blind, Julia et al**
**Avantcore Rechtsanwälte Partnerschaft mbB**
**Hohenzollernstraße 1**
**70178 Stuttgart (DE)**

(56) References cited:
**WO-A1-98/32107**  **WO-A1-2011/063248**
**US-A- 5 397 865**  **US-A1- 2012 035 498**
**US-A1- 2014 045 148**

**Description**

BACKGROUND

The invention relates to a computer-implemented method and system for testing and/or training visual, cognitive and/or eye-hand coordination functions or skills of a user according to independent claim 1. WO 2008/128192 A1 discloses a computer-implemented method for testing and training of vision, cognitive function, and eye-hand coordination based on a variety of stimuli provided to a user. The corresponding responses received from the user may depend upon the combination of the stimuli. To provide the stimuli and to receive responses, a touch screen display is used wherein behavioral information regarding the user's performance is recorded and wherein a scoring is based upon the speed, accuracy of the user's touch response, and other aspects of the user's performance, is done. In particular, an object is displayed at a first time and at a first location of the touch-sensitive display and a touch input from the user at a second time and at a usually varying second location is received, wherein determining the point-to-point distance between the two locations and the time elapsed between the first time and the second time.

WO 2013/111746 A1 discloses a computer-implemented method and system for testing cognitive functions related to e.g. Attention Deficit Hyperactivity Disorder (ADHD) which enable to estimate the user's probability having a medical disorder such as ADHD. More particularly, a problem is displayed on a touch panel display, wherein the user responds by manipulating the touch display and wherein the user's responses are recorded in a problem/answer table. An estimation calculation based on a learning algorithm is used to estimate the degree of proper operation of the user's cognitive functions. The documents US 2012/035498 A1, WO 2011/063248 A1, US5 397 865 A, WO 98/32107 A1 and US 2014/045148 A1 also each disclose computer-implemented methods for testing and training visual, cognitive and eye-hand coordination functions of a subject, by analysing the similarity between a line-shaped object that has been originally presented on a touch-sensitive display and a re-traced object by the subject.

SUMMARY

The herein disclosed computer-implemented method and system for testing and/or training visual, cognitive and/or eye-hand coordination functions or skills of a user is based on the concept of determining or evaluating conformity or deviation of a manually traced or retraced line-shaped object, structure or pattern, wherein the object, structure or pattern is presented by the computer at least temporarily and the user input is gathered using a graphical input device like a touch panel display or a graphic tablet.

In contrast to the above mentioned known approaches, according to the present invention, line-shaped, i.e. straight or curved objects, structures or patterns are presented to the user wherein the user has to trace or retrace the trajectory of the presented structure(s) or pattern(s) as accurate as possible. The retraced object(s), structure(s) or pattern(s) entered by the user is (are) evaluated in order to determine line-based deviations and/or conformity thus enabling to calculate a characteristic number or parameter. Based on the calculated number or parameter, the user's visual, cognitive and/or eye-hand coordination functions can be assessed in a highly reproducible way so that significant or valid results of a medical assessment are being made possible.

In particular, evaluation of the user's input can reveal a scalar or multi-dimensional characteristic number or parameter which is used to determine the user's visual, cognitive and/or eye-hand coordination skills thus providing rather characteristic and thus relevant information to assess the user's health status.

Evaluation or determination of conformity/deviations is preferably based on linear or even non-linear summation or integration of gathered deviation values. In case of linear summation/integration, a scalar number (mentioned characteristic number) can be calculated fast which, thereupon, can be further processed with relatively small technical efforts. The invention thus allows for an immediate feedback to the user which advantageously enhances the usefulness of the inventive approach, in particular when being used as a training tool.

In addition to the evaluation of conformity/deviations, the required time to retrace a complete object, structure, or pattern or a part of it, e.g. the time elapsed between drawing two lines of such an object, structure or pattern, can be evaluated, i.e. compared with provided corresponding data of e.g. test patients having a certain disease. Also, a relative shift or displacement of the position of an object, structure or pattern being drawn (retraced) by the user, in relation to the position of the presented one, can be taken into consideration, in order to enhance significance and/or validity of the evaluation results, in particular in view of the user's eye-hand coordination functions or skills.

The presented and to be retraced objects, structures or patterns can be one-dimensional, two-dimensional or even virtually three-dimensional. The only restriction is that they have to be line-shaped in order to allow for the mentioned evaluation of conformity, or deviations respectively. The line shapes preferably comprise lines of at least two directions, e.g. a mentioned basic mathematic shape or e.g. a cross, so that the user has to change the hand's moving direction when drawing (retracing) a presented line shape. Change of the moving direction can be used advantageously to test or train the user's eye-hand coordination skills. In addition, such two-dimensional objects can be alphabetic or calligraphic characters, in the latter case e.g. Japanese Kanji, Hiragana or Katakana characters.

In addition to the mentioned evaluation of conformity/deviations, the order or sequence of the user's entering the lines of (a) retraced object(s), structure(s) or pattern(s) can be evaluated. Such information can be used to obtain a better understanding of the user's cognitive functions. Beyond that, also the line width or the pressure being applied by the user to a graphical input device like a touch screen pen or a (the) user's finger(s) can be evaluated for the same purpose.

In addition to, or instead of, retracing a line-shaped object, structure or pattern, the user may have to color the inside of a certain line-shaped object, structure or pattern, wherein the user's drawing is evaluated in view of conformity of the coloring with the border lines of the object. Such an approach can be used advantageously when testing or training users with artistic interests or younger people.

By presenting at least partially hidden or masked objects, structures or patterns, for instance presenting only some supporting points or nodes for these objects etc., the user's cognitive functions can be tested or trained more effectively. As supporting points, e.g. two or three points can be presented in order to draw a half circle which is clearly determined by these three points. However, only two points for drawing a circle can be used in the case that these points are located at diametrically opposed sides of the circle.

Furthermore, the object(s), structure(s) or pattern(s) can be presented only temporarily and thus have to be retraced by the user based on the user's short-term memory, wherein the user's input is evaluated like with a human brain memory test.

The calculated characteristic number or parameter being characteristic of the user's visual, cognitive and/or coordination functions can be used either for training purposes and/or to assess the user's health condition or status, in particular in view of the mentioned possible ADHD syndrome, possible movement disorders e.g. in case of a Parkinson's disease, or any other similar disease, damage or malfunction of a human's brain or body.

## BRIEF DESCRIPTION OF THE DRAWINGS

The objects, features and advantages of the invention are understood within the context of the Detailed Description, as set forth below. The Detailed Description is understood within the context of the accompanying drawings, which form a material part of this disclosure, wherein:

FIG.s 1A - C      depict an enlarged view of a computer-generated, pixel-oriented circular shape, together with a user's retraced line, in order to illustrate the method according to the invention;

FIG.s 2A, B      depict an overview and enlarged view of a computer-generated arbitrary shape, together with a user's coloring inside the shape, according to an embodiment of the invention;

FIG.s 3 - 8      shall illustrate different application scenarios of the invention;

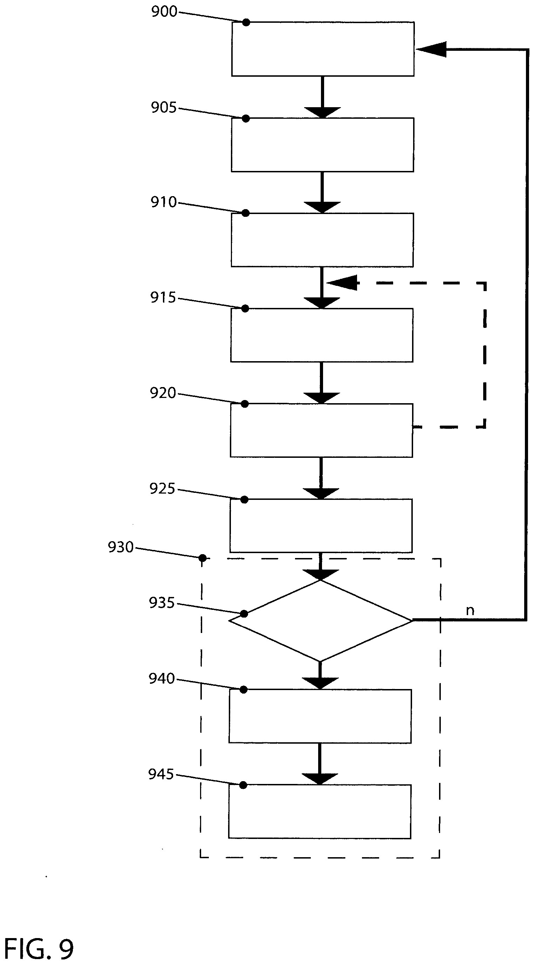

FIG. 9      is a flow diagram of a preferred embodiment of the method according to the invention.

## DETAILED DESCRIPTION

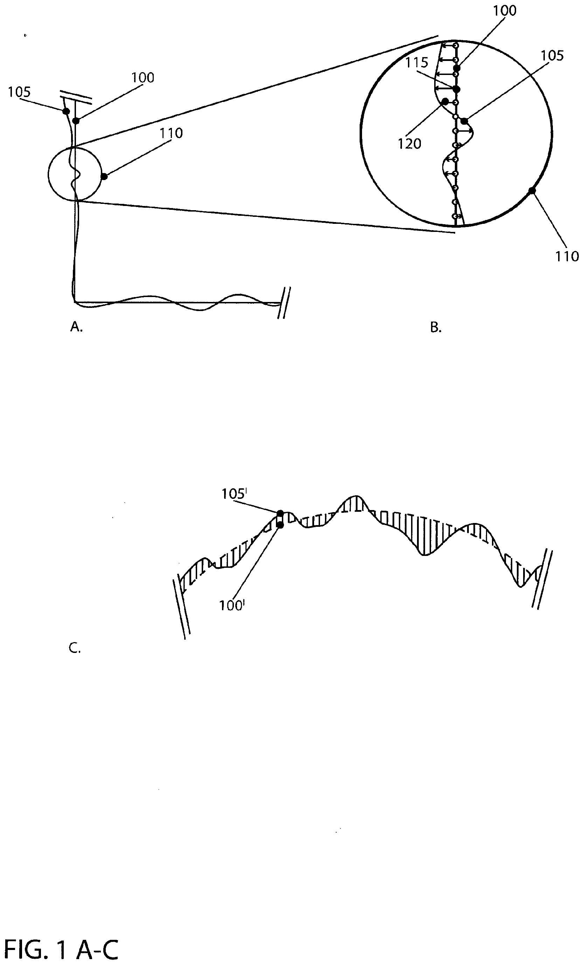

FIGURES 1A und 1B show a corner section (FIGURE 1A) and an enlarged view (FIGURE 1B) of a pixel-oriented quadratic or rectangular shape (line 100 in FIGURE 1A) being presented on a touch-sensitive display or screen of an underlying digital processing or computer device which has been re-drawn or re-traced according to line 105 by a user of the device. It can be seen that the retraced line 105 deviated from the presented line 100 wherein the deviation is highlighted by means of hatching lines.

For the part within the circle 110 shown in FIGURE 1A, an enlarged or zoomed view is depicted in FIGURE 1B. The mentioned pixels are shown as dots 115 in FIGURE 1B. The deviation between each pixel and a corresponding point on the re-traced line are depicted as vectors 120. It can be seen that these vectors 120 comprise different signs depending on the direction of an underlying deviation.

Only for illustrative purposes, FIGURE 1C shows another shape 100' and a corresponding re-traced line 105', namely a section of a circular shape. As in the previous example, the deviation is highlighted by hatching lines.

Based on the above described deviations or vectors 120, the following algorithm, in the present embodiment a linear equation, is used to calculate, as a characteristic number, the integral value of all deviations along the re-traced line 105, 105'. The characteristic number in this example is scalar but can be multi-dimensional, e.g. a vector or matrix, in case of multi-dimensional equation system being used instead of the present linear equation. The described algorithm provides for an automated calculation of the conformity or deviation between an arbitrary presented shape and a re-drawn or re-traced line or shape. Thus advantageously, an immediate feedback by the system to the user is possible.

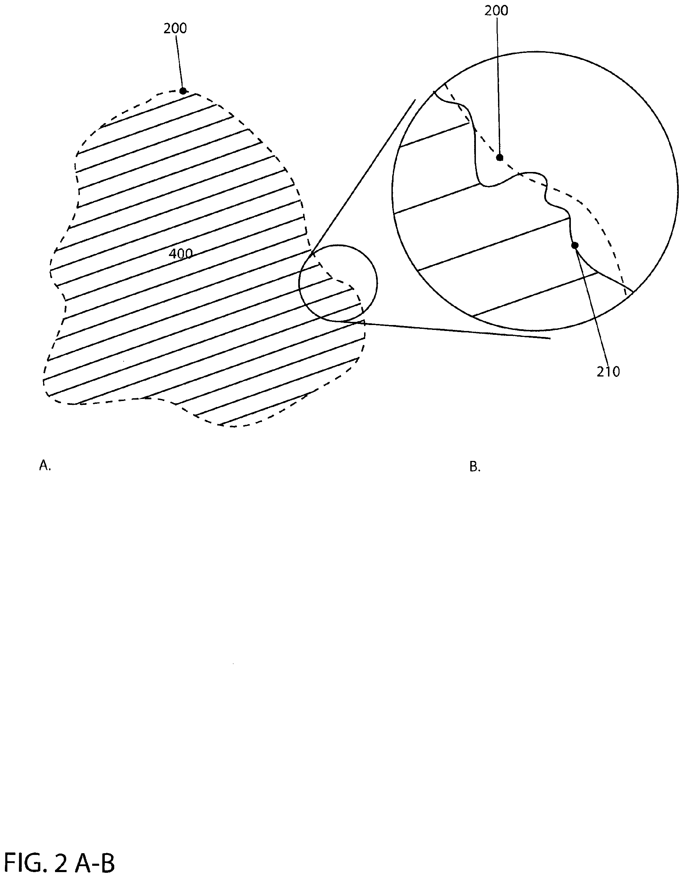

FIGURES 2A and 2B show an embodiment of a shape 200, together with an enlarged view of the shape's border, wherein a user has to color or fill in the inside area 205 of the shape using a touch pen or a user's finger. As in the previous example, the user's coloring will not be perfectly aligned with the shape's border thus causing deviations between the shape's border 200 being presented by the computer and the border line 210 of the coloring.

The above illustrated deviations or conformity between a presented shape and a retraced line or coloring drawn by the user, in the present embodiment, will be evaluated based on the following described calculation steps and corresponding mathematical formulas.

The calculation of a drawing's key quantity, compared to the presented original, is based upon the following modular steps, where it is assumed that a drawing consists of $x * y = n$ pixels (P) and $m$ drawing points (DP), with $DP \subset P$.

- Step 1: Average pixel distance (apd)

$$apd = \frac{\sum_{i=1}^{n} distance(P(i)_{drawing}, P(i)_{original})}{n} \tag{1}$$

The average pixel distance sets the average number of pixels that a drawn pixel is away from the nearest original pixel where a bigger distance leads to higher values. It holds information about how close the (re-)drawn lines are to the original, but gives no information about how much of the original lines are covered.

- Step 2: Average variance (var)

$$var = \frac{\sum_{i=1}^{n}(distance(P(i)_{drawing}, P(i)_{original}) - apd)^2}{n} \tag{2}$$

The average variance holds information about how consistently the drawing is made (see Fig. 1A-C). In most cases, a varying distance between the (re-)drawn and the original line leads to higher values.

- Step 3: Curve length (cl) and curve length difference (cld)

$$cl = \sum_{i=1}^{m-1} \sqrt{(DP(x)_{i+1} - DP(x)_i)^2 + (DP(y)_{i+1} - DP(y)_i)^2} \tag{3}$$

$$cld = |cl_{drawing} - cl_{original}|$$

Based upon the drawing points, the curve length is calculated to be compared to the original curve length. A bigger difference between drawing and original leads to higher values of cld.

- Step 4: Average inverse pixel distance (ipd)

$$ipd = \frac{\sum_{i=1}^{n} distance(P(i)_{original}, P(i)_{drawing})}{n} \tag{4}$$

The average inverse pixel distance sets the average number of pixels that an original pixel is away from the nearest drawn pixel where a bigger distance leads to higher values. It holds information about how close the original lines are to the drawn ones and thereby gives information about how much of the original lines are covered.

- Step 5: The time needed to draw

$$time = seconds\ needed \tag{5}$$

The amount of time which is needed to perform the drawing is measured in seconds.

For calculating the key quantity, quotients based upon these values (1) - (5) are built or calculated which transcribe them to values between values 0 and 100, wherein a value of 0 means a big difference between the original and the drawing curves or lines, and wherein a value of 100 means an essentially perfect score. For this, additional ,worst' and ‚best' values are taken into account for every quotient (1) - (5) as follows:

$$apd_{best} = 1, \; apd_{worst} = 72$$

$$var_{best} = 1{,}5, \; var_{worst} = 15$$

$$cld_{best} = 10, \; cld_{worst} = cl_{original}$$

$$ipd_{best} = 0, \; ipd_{worst} = 128$$

$$time_{best} = 1, \; time_{worst} = 24 \tag{6}$$

wherein, based on these value pairs (6), calculating the following modular quotients:

$$q_{apd} = \min_{100}(\max_{0} 100 \frac{apd_{worst} - apd}{apd_{worst} - apd_{best}})$$

$$q_{var} = \min_{100}(\max_{0} 100 \frac{var_{worst} - var}{var_{worst} - var_{best}})$$

$$q_{cld} = \min_{100}(\max_{0} 100 \frac{cld_{worst} - cld}{cld_{worst} - cld_{best}})$$

$$q_{ipd} = \min_{100}(\max_{0} 100 \frac{ipd_{worst} - ipd}{ipd_{worst} - ipd_{best}})$$

$$q_{time} = \min_{100}(\max_{0} 100 \frac{time_{worst} - time}{time_{worst} - time_{best}}) \tag{7}$$

Based upon these modular quotients (7), the key quantities for different approaches can now be calculated. Taking all quotients (7) into account, the two main scores are an average score (8) and a multiplicative score (9), as follows:

$$score_{avg} = \frac{q_{apd} + q_{var} + q_{cld} + q_{ipd} + q_{time}}{5} \tag{8}$$

$$score_{mult} = 100 \left( \frac{q_{apd}}{100} \frac{q_{var}}{100} \frac{q_{cld}}{100} \frac{q_{ipd}}{100} \frac{q_{time}}{100} \right) \tag{9}$$

Generally speaking, the multiplicative score is somewhat stronger and thus it is required to be more accurate to get as high results as with the average score.

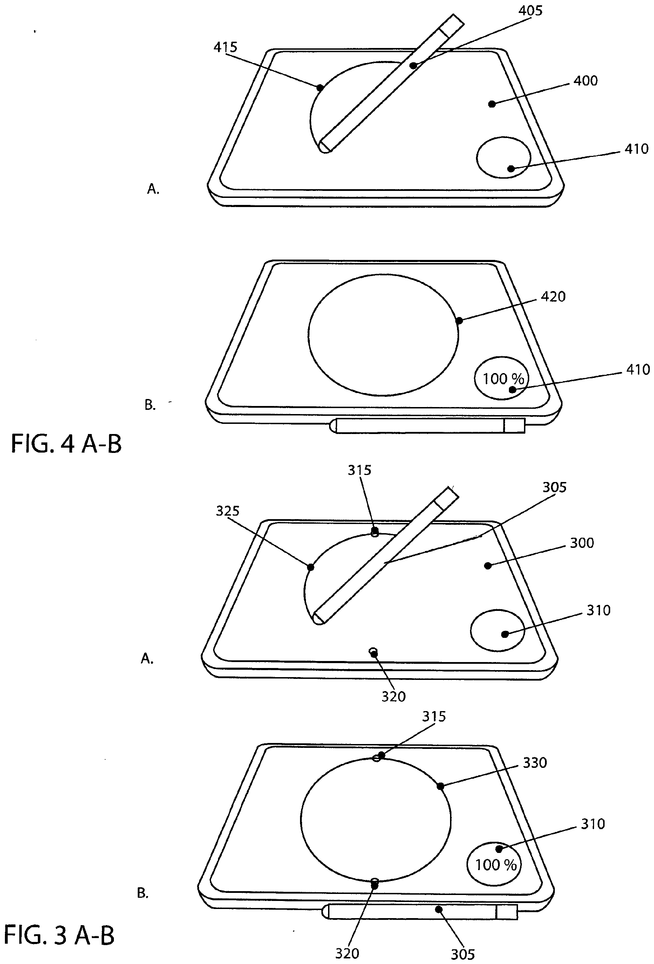

The present invention can be used in various different application scenarios, examples or embodiments of which are described in the following, referring to FIGURES 3 to 8.

Referring to the embodiment illustrated in FIGURES 3A and 3B, a touchpad 300 of a portable computer is shown where the user input is accomplished using a touch pen 305. The two FIGURES 3A and 3B illustrate this action or process at two consecutive time stages. Of course, user input can also be accomplished using a user's finger. The area 310 on the touch screen 300 provides information to the user regarding conformity or deviation of the user's input in relation to a presented object or structure. In this and the following embodiments, the presented information is the conformity of a user's input with a presented object or structure, namely in units of percentage [%].

In general, the presented and to be redrawn object(s) or structure(s) are basic mathematic shapes like one or more circles, squares, rectangles, or triangles. In case of one circle being presented to the user, three or more supporting points are required to clearly define the expected shape and thus at least three points are presented which have to be crossed or cut by the re-drawn (retraced) circle. In case of a square, accordingly, three supporting points to be located somewhere on different lines of the square are required to clearly define the expected shape and thus at least three points are presented which have to be crossed or cut by the drawn (retraced) square. In case of a triangle, accordingly, three supporting points to be located at different lines of the triangle are required to clearly define the expected shape and thus at least three points are presented which have to be crossed or cut by the drawn (retraced) triangle.

Alternatively, instead of presenting the mentioned required number (in the above cases three) of supporting points, in the present embodiment, only two points 315, 320 are presented asking the user to draw e.g. a circle 325, 330 wherein the two supporting points 315, 320 in this embodiment define the circle's diameter or may be located somewhere on the circle thus allowing the user to draw a circle with an arbitrary diameter. Accordingly, a square can be clearly defined by only two supporting points if they represent opposing corners of the square. In case of a triangle, the user can be asked to draw an equal-sided triangle based on only two presented points. As illustrated by the embodiment shown in FIGURES 4A and 4B, which also depicts a touch screen 400, pen 405 and information area 410, a circle 415 or the like can even be drawn free-hand by the user without any supporting points, and thus also with an arbitrary diameter. In the two cases where a circle or the like is drawn with an arbitrary diameter (or size, in cases of other regular shapes like a square), the final shape 420 drawn by the user is compared with a corresponding shape which is calculated e.g. by a least-square matching calculation ("least square fit").

The above described method can be advantageously used in the fields of human medical diagnosis and/or therapy. Now referring to an embodiment not shown in a figure, the presented samples of object(s), structure(s) or pattern(s) comprise preferably standardized shapes for which other patient's data, e.g. data of test patients, are available, e.g. patients suffering from an apoplectic stroke ("Apoplexia") or any other brain damage who have a reduced ability to draw line-shaped objects etc. Based on the method and system according to invention, such patients advantageously can perform therapeutic exercises without the attendance of medical personnel and particularly obtain the positive results of such exercises without any delay thus motivating them to continue with these exercises.

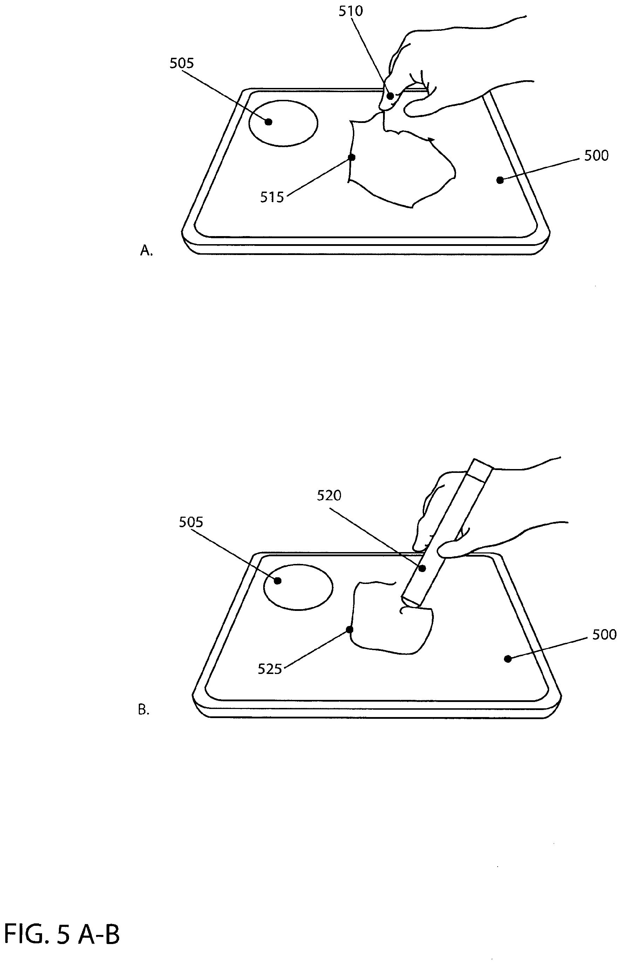

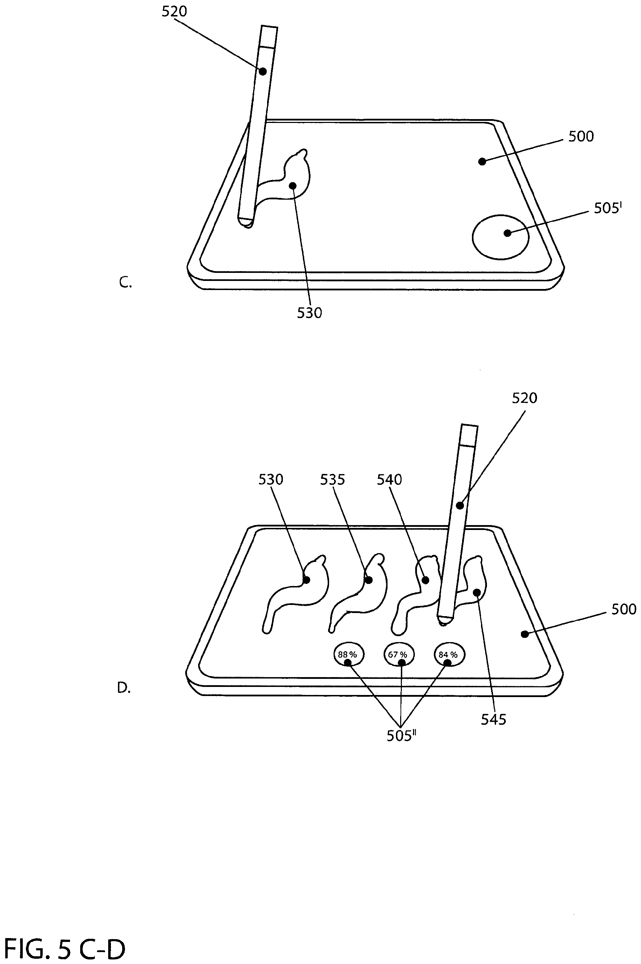

Referring to the embodiment illustrated in FIGURES 5A and 5B, which again depicts a touch screen 500 and an information area 505, wherein the user input, in FIGURE 5A, is accomplished using a user's finger 510, and in FIGURE 5B, using a digitizer or pen 520. The presented sample(s) of object(s), structure(s) or pattern(s) are not generated by the computer but the user him-/herself. In this embodiment, the user first draws an arbitrary line-shaped object, structure or pattern 515, 525 which is stored by the computer. Such a drawn object, structure or pattern is used as a kind of master and will be compared with (an) successively drawn shapes of (an) as much as possible identical object(s), structure(s) or pattern(s). Evaluation of conformity or deviation between successively drawn shapes 530, 535, 540, 545 can be used to determine the user's cognitive status in view of repeating events, as illustrated in FIGURES 5C and 5D, e.g. his/her power of concentration. The determined user's cognitive status, in the present embodiment, is presented to the user by means of different information areas 505", each of them being related to one of the shapes 535, 540, 545. Such an embodiment thus can be used to detect a mentioned ADHD syndrome. As in some of the herein described other embodiments, other parameters like the time required to retrace an object or part of an object, the applied pressure when drawing an object or part of an object, the order of drawing lines, or the direction of drawing lines can be detected or monitored thus enhancing the validity of the evaluation results.

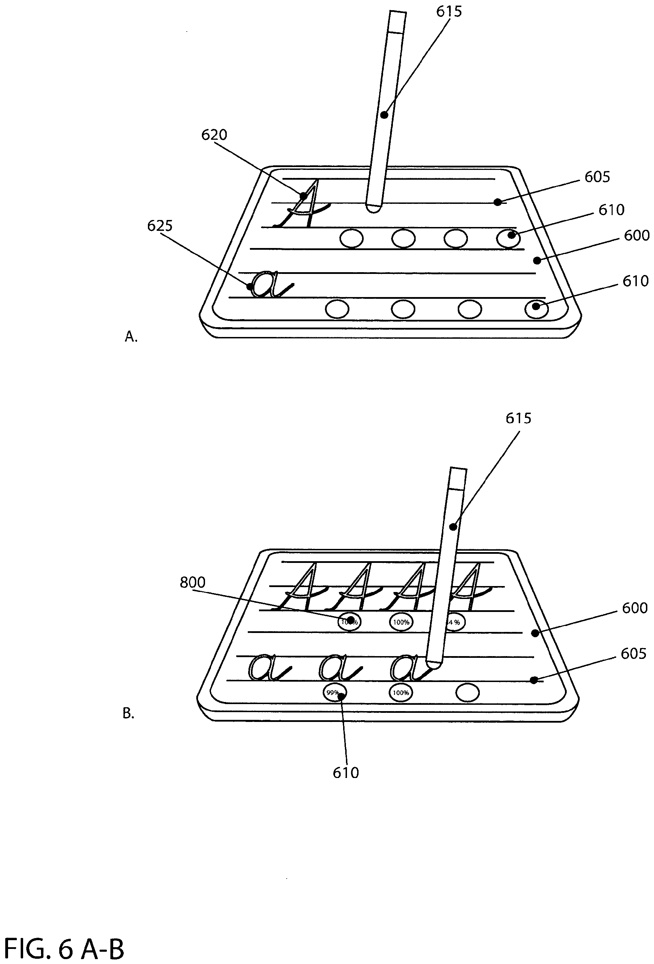

Now referring to the embodiment illustrated in FIGURES 6A and 6B, which again shows a touch screen 600 with horizontal (auxiliary) drawing lines 605, several information areas 610 and a user's input pen 615. The objective in the present scenario is to teach a language alphabet. In this embodiment, the presented object(s) is (are) language characters. In the present embodiment, sample characters for upper case 620 and lower case letters 625 are presented by the computer on the left side of the touch screen 600 and the user draws or enters the character at a different position of the screen. In the present embodiment, the two distinct user entry areas allow a user to redraw both upper and lower case letters. However, this is only a certain application scenario, and thus two or even more entry areas or fields can be used for learning purposes as well, or any other purpose. The above described fast algorithm for evaluation of the entered characters allows for an immediate feedback which provides enhanced motivation to the user to continue with the learning exercises.

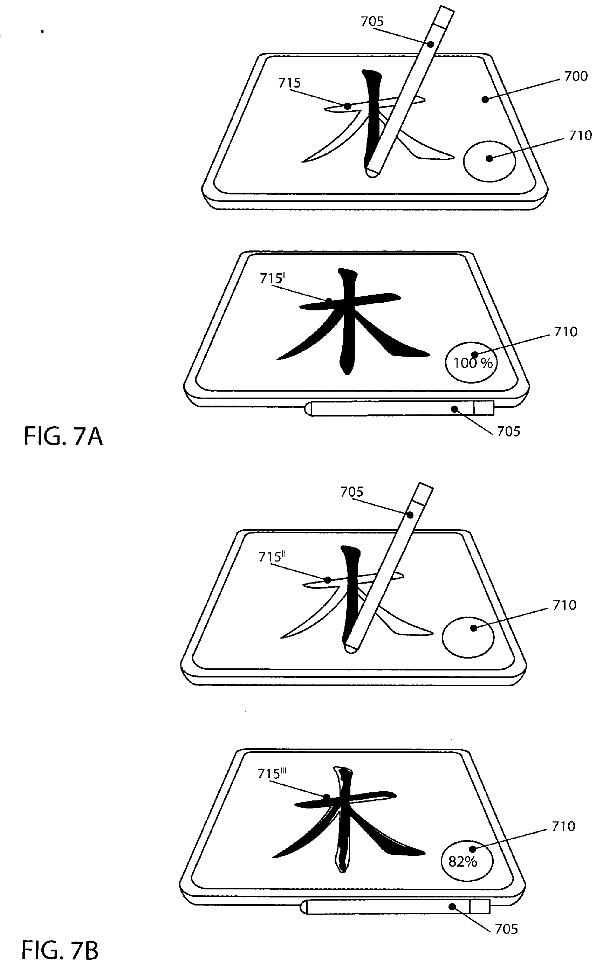

Referring to the embodiment illustrated in FIGURES 7A and 7B, again showing a touch screen 700, an input pen 705 and an information area 710, the object(s) or structure(s) are letters or characters 715 being used for calligraphy, e.g. those being used in Japanese language. The particular aspect of such characters is the varying line width and with real drawing of such characters on paper thus required different pressure to be applied by the user to the pen when drawing the character 715', 715", 715'''. Accordingly, in this embodiment, the user is asked to retrace a presented character wherein varying the pressure applied to the input device, or the user's finger if being used as input device. In addition, when drawing Japanese characters it is essential to consider the right order of lines to be drawn. Insofar, evaluation of conformity or deviations between the presented character and the retraced character takes the line width and order of drawing the lines into consideration. It is emphasized that the final drawing does not show the order of drawing lines but using a touch screen, graphic tablet or the like enables to monitor and/or to protocol the order of entered lines. In the present embodiment, areas of the drawn (retraced) character which are not conform or congruent with the presented character (see e.g. 715'''), are highlighted or marked, e.g. using a certain color scheme or the like.

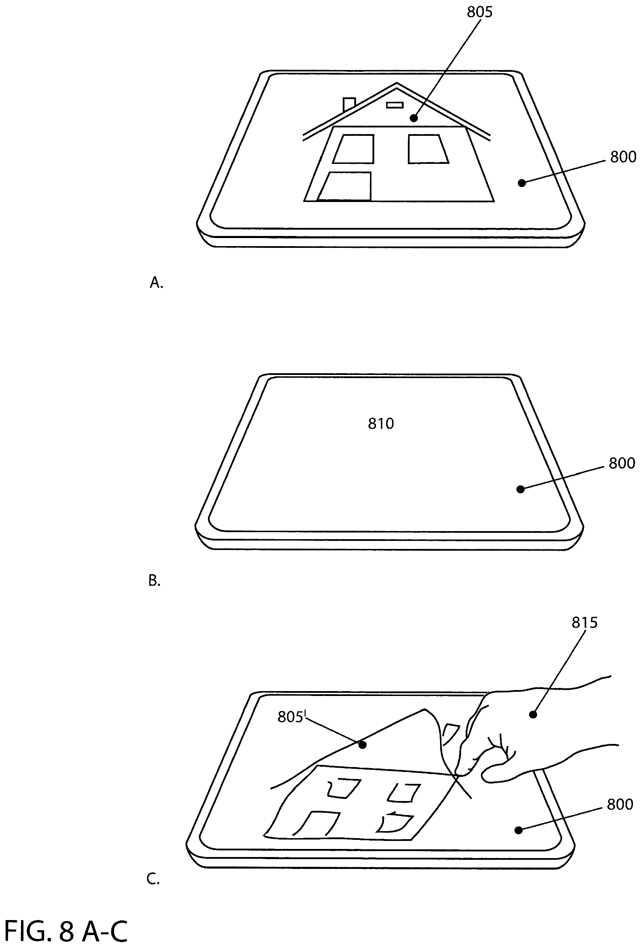

Referring to another embodiment illustrated in FIGURES 8A to 8C, showing three time stages of a process according to the invention using again a touch screen 800, the samples of object(s), structure(s) or pattern(s) are presented only temporarily, i.e. only during the first stage (FIGURE 8A) 805 and then disappearing as illustrated by the blank screen 810 during the second stage (FIGURE 8B), and thus have to be redrawn or retraced 805' by the user during the third stage (FIGURE 8C), in the present embodiment by a user's finger 815, based on the user's short-term memory, wherein the user's input is evaluated like with a memory test. This embodiment particularly allows for testing (assessing) or training the user's capacity for remembering.

Now referring to the flow diagram depicted in FIGURE 9, according to the first step 900 of the shown routine, a pixel- or vector-oriented shape to be presented on a touch screen is generated. The generated shape is then presented 905 on the touch screen. In step 910 any user input is detected and the corresponding pixel coordinated being stored at least intermediately. Of course, step 910 thus may consist of a number of recurring single steps which are implemented to detect the user input on a discrete i.e. pixel-oriented basis.

In step 915 of the shown routine, the stored pixels of the user input are compared with corresponding pixels of the computer-presented shape. This comparison reveals deviation values which are summed-up or integrated in step 920. As illustrated by the dashed arrow line, this summing-up can be done on a recurring pixel-per-pixel basis until all pixels have been considered. As described above, the summing-up can be performed either using the measured deviation values including their signs, thus revealing a final score which indicates the relative conformity of the redrawn shape with regard to the center of the presented line shape, or using absolute deviation values, thus providing indication of the overall conformity of the redrawn shape with the presented shape. After all pixels have been processed, the finally calculated score is presented 925 to the user, e.g. using a pre-described information field 310.

According to the further optional steps (separated by dashed line 930), the first step 900 has to be modified in order to generate at least two shapes n wherein n = 1, ..., $n_{max}$, with $n_{max}$ being the number of (different) shapes to be generated by the computer. By means of test step 935 which checks if the mentioned maximum number of shapes $n_{max}$ to be presented has been reached (i.e. the condition $n > n_{max}$ is fulfilled), the previously described steps 900 - 925 are repeated until this condition is fulfilled. According to following step 940, all single scores being calculated in step 920 are used to calculate a final score or to provide an assessment about the likelihood that the user is suffering from a certain disease, in particular a mentioned brain disease. The final score or medical assessment is then presented 945 to the user, e.g. by way of an information or text box being presented on the touch screen.

The herein described method and system can be implemented in a medical or training application or appliance running on any computer or data processing system having a touch screen, like a personal (stationary) computer, a portable computer like a laptop or tablet computer, or mobile devices with a touch screen like smart phones.

**Claims**

1. A computer-implemented method for testing and/or training visual, cognitive and/or eye-hand coordination functions or skills of a user, using a computer or data processing device having a touch-sensitive display, wherein at least one line-shaped object (100) is presented on the touch-sensitive display, wherein the user has to retrace the line(s) of the presented object (100) and wherein the retraced line(s) (105) is (are) evaluated in order to determine a characteristic which relates to deviations and/or conformity (120) between the presented at least one line-shaped object (100) and the retraced line(s) (105), wherein the characteristic is determined based on evaluating the time required for retracing the line(s) of the presented object, **characterized in that** the characteristic is furthermore determined based on the following evaluation steps:

   Evaluating an average pixel distance for pixels that are drawn away from a nearest originally presented pixel; evaluating an average variance that holds information about how consistently the drawing is made; evaluating a curve length in comparison with an original curve length; evaluating an average inverse pixel distance that holds information about how far an original pixel is away from the nearest drawn pixel.

2. Method according to claim 1, wherein the determined characteristic is used to assess the user's visual, cognitive and/or eye-hand coordination functions, or the user's health status.

3. Method according to claim 1 or 2, wherein the characteristic is a scalar number or a multi-dimensional number.

4. Method according to any of the preceding claims wherein the characteristic is determined by a linear or non-linear summation or integration of single deviation values.

5. Method according to any of the preceding claims wherein the time required for retracing the line(s) of the presented object is evaluated.

6. Method according to any of the preceding claims, wherein calculating the average pixel distance (apd) is based on the following equation (1)

$$apd = \frac{\sum_{i=1}^{n} distance(P(i)_{drawing}, P(i)_{original})}{n} \qquad ; \qquad (1)$$

wherein calculating the average variance (var) is based on the following equation (2)

$$var = \frac{\sum_{i=1}^{n}(distance(P(i)_{drawing}, P(i)_{original}) - apd)^2}{n} \qquad ; \qquad (2)$$

wherein calculating the curve lenath (cl) and an according curve length difference (cld) is based on the following two equations (3)

$$cl = \sum_{i=1}^{m-1} \sqrt{(DP(x)_{i+1} - DP(x)_i)^2 + (DP(y)_{i+1} - DP(y)_i)^2}$$

$$cld = |cl_{drawing} - cl_{original}|; \qquad (3)$$

wherein calculating the average inverse pixel distance (ipd) is based on the following equation (4)

$$ipd = \frac{\sum_{i=1}^{n} distance(P(i)_{original}, P(i)_{drawing})}{n} \qquad ; \qquad (4)$$

wherein the time needed for retracing the line(s) of the presented object is measured in seconds.

7. Method according to claim 6 wherein the following modular quotients are calculated:

$$q_{apd} = \min_{100}(\max_{0} 100 \frac{apd_{worst} - apd}{apd_{worst} - apd_{best}})$$

$$q_{var} = \min_{100}(\max_{0} 100 \frac{var_{worst} - var}{var_{worst} - var_{best}})$$

$$q_{cld} = \min_{100}(\max_{0} 100 \frac{cld_{worst} - cld}{cld_{worst} - cld_{best}})$$

$$q_{ipd} = \min_{100}(\max_{0} 100 \frac{ipd_{worst} - ipd}{ipd_{worst} - ipd_{best}})$$

$$q_{time} = \min_{100}(\max_{0} 100 \frac{time_{worst} - time}{time_{worst} - time_{best}})$$

**8.** Method according to claim 7 wherein, based upon the modular quotients, a main score is calculated as an average score (score$_{avg}$) or a multiplicative score (score$_{mult}$), as follows:

$$score_{avg} = \frac{q_{apd} + q_{var} + q_{cld} + q_{ipd} + q_{time}}{5}$$

or

$$score_{mult} = 100 \left( \frac{q_{apd}}{100} \frac{q_{var}}{100} \frac{q_{cld}}{100} \frac{q_{ipd}}{100} \frac{q_{time}}{100} \right).$$

**9.** Method according to any of claims 5 to 8 wherein the time elapsed between drawing two lines of the presented object is evaluated, in particular wherein said elapsed time is compared with provided corresponding data of test patients having a certain disease.

**10.** Method according to any of the preceding claims wherein a relative displacement of the position of a retraced object, in relation to the position of the presented object is evaluated.

**11.** Method according to any of the preceding claims wherein the lines of the presented object are retraced using at least two moving directions.

**12.** Method according to any of the preceding claims wherein the order of the retraced lines is evaluated.

**13.** Method according to any of the preceding claims wherein the line width and/or the pressure being applied by the user when retracing an object are evaluated.

**14.** Method according to any of the preceding claims, wherein the inside of a presented line-shaped object has to be colored by the user and wherein conformity of the coloring with the border lines of the presented object is evaluated.

**15.** Method according to any of the preceding claims wherein an at least partially hidden or masked line-shaped object is presented.

**16.** Method according to claim 15 wherein at least two supporting points of a presented object are presented.

**17.** Method according to any of the preceding claims wherein an object is presented only temporarily.

**18.** A computer-implemented system for testing and/or training visual, cognitive and/or eye-hand coordination functions or skills of a user, wherein the system comprises a touch-sensitive display, **characterized by** processing means for executing the method according to any of the preceding claims.

**19.** System according to claim 18 comprising processing means for presenting at least one line-shaped object (100) on the touch-sensitive display, for detecting information about line(s) of the presented object (100) being retraced (105) by the user, using the touch-sensitive display, and for evaluating the detected information in order to determine a characteristic which relates to deviations and/or conformity (120) between the presented at least one line-shaped object (100) and the retraced line(s) (105).

**Patentansprüche**

**1.** Computerimplementiertes Verfahren zum Testen und/oder Trainieren visueller, kognitiver und/oder Auge-Hand-Koordinationsfunktionen oder -fähigkeiten eines Benutzers unter Verwendung einer Computer- oder Datenverarbeitungseinrichtung mit einem berührungsempfindlichen Display, wobei mindestens ein linienförmiges Objekt (100) auf dem berührungsempfindlichen Display vorgelegt wird, wobei der Benutzer die Linie(n) des vorgelegten Objekts (100) nachziehen soll und wobei die nachgezogene Linie (n) (105) evaluiert wird/werden, um eine Charakteristik zu bestimmen, die Abweichungen und/oder eine Konformität (120) zwischen dem vorgelegten mindestens einen linienförmigen Objekt (100) und der/den nachgezogenen Linie(n) (105) betrifft, wobei die Charakteristik auf der Basis des Evaluierens der für das Nachziehen der Linie(n) des vorgelegten Objekts erforderlichen Zeit bestimmt wird, **dadurch gekennzeichnet, dass** die Charakteristik weiterhin auf der Basis der folgenden Evaluierungsschritte bestimmt wird:

Evaluieren einer mittleren Pixeldistanz für Pixel, die von einem nächstgelegenen, ursprünglich vorgelegten Pixel weg gezeichnet werden;
Evaluieren einer mittleren Varianz, die Informationen darüber enthält, wie konsistent die Zeichnung gemacht wird;
Evaluieren einer Kurvenlänge im Vergleich mit einer ursprünglichen Kurvenlänge;
Evaluieren einer mittleren inversen Pixeldistanz, die Informationen darüber enthält, wie weit ein ursprüngliches Pixel von dem nächstgelegenen gezeichneten Pixel weg liegt.

2. Verfahren nach Anspruch 1, wobei die bestimmte Charakteristik verwendet wird zum Beurteilen der visuellen, kognitiven und/oder Auge-Hand-Koordinationsfunktionen des Benutzers oder des Gesundheitszustands des Benutzers.

3. Verfahren nach Anspruch 1 oder 2, wobei die Charakteristik eine skalare Zahl oder eine mehrdimensionale Zahl ist.

4. Verfahren nach einem der vorhergehenden Ansprüche, wobei die Charakteristik durch eine lineare oder nichtlineare Summierung oder Integration einzelner Abweichungswerte bestimmt wird.

5. Verfahren nach einem der vorhergehenden Ansprüche, wobei die zum Nachziehen der Linie (n) des vorgelegten Objekts erforderliche Zeit evaluiert wird.

6. Verfahren nach einem der vorhergehenden Ansprüche, wobei das Berechnen der mittleren Pixeldistanz (apd) auf der folgenden Gleichung (1) basiert

$$apd = \frac{\sum_{i=1}^{n} distance(P(i)_{drawing}, P(i)_{original})}{n} ; \qquad (1)$$

wobei das Berechnen der mittleren Varianz (var) auf der folgenden Gleichung (2) basiert

$$var = \frac{\sum_{i=1}^{n}(distance(P(i)_{drawing}, P(i)_{original}) - apd)^2}{n} ; \qquad (2)$$

wobei das Berechnen der Kurvenlänge (cl) und einer entsprechenden Kurvenlängendifferenz (cld) auf den folgenden beiden Gleichungen (3) basiert

$$cl = \sum_{i=1}^{m-1} \sqrt{(DP(x)_{i+1} - DP(x)_i)^2 + (DP(y)_{i+1} - DP(y)_i)^2}$$

$$cld = | cl_{drawing} - cl_{original} |; \qquad (3)$$

wobei das Berechnen der mittleren inversen Pixeldistanz (ipd) auf der folgenden Gleichung (4) basiert

$$ipd = \frac{\sum_{i=1}^{n} distance(P(i)_{original}, P(i)_{drawing})}{n} ; \qquad (4)$$

wobei die zum Nachziehen der Linie (n) des vorgelegten Objekts benötigte Zeit in Sekunden gemessen wird.

7. Verfahren nach Anspruch 6, wobei die folgenden modularen Quotienten berechnet werden:

$$q_{apd} = \min_{100}(\max_{0} 100 \frac{apd_{worst} - apd}{apd_{worst} - apd_{best}})$$

$$q_{var} = \min_{100}(\max_{0} 100 \frac{var_{worst} - var}{var_{worst} - var_{best}})$$

$$q_{cld} = \min_{100}(\max_{0} 100 \frac{cld_{worst} - cld}{cld_{worst} - cld_{best}})$$

$$q_{ipd} = \min_{100}(\max_{0} 100 \frac{ipd_{worst} - ipd}{ipd_{worst} - ipd_{best}})$$

$$q_{time} = \min_{100}(\max_{0} 100 \frac{time_{worst} - time}{time_{worst} - time_{best}}).$$

8. Verfahren nach Anspruch 7, wobei auf der Basis der modularen Quotienten ein Hauptscore als ein mittlerer Score ($score_{avg}$) oder ein multiplikativer Score ($score_{mult}$) wie folgt berechnet wird:

$$score_{avg} = \frac{q_{apd} + q_{var} + q_{cld} + q_{ipd} + q_{time}}{5}$$

oder

$$score_{mult} = 100 \left( \frac{q_{apd}}{100} \frac{q_{var}}{100} \frac{q_{cld}}{100} \frac{q_{ipd}}{100} \frac{q_{time}}{100} \right).$$

9. Verfahren nach einem der Ansprüche 5 bis 8, wobei die zwischen dem Zeichnen von zwei Linien des vorgelegten Objekts verstrichene Zeit evaluiert wird, wobei insbesondere die verstrichene Zeit mit bereitgestellten entsprechenden Daten von Testpatienten mit einer gewissen Krankheit verglichen wird.

10. Verfahren nach einem der vorhergehenden Ansprüche, wobei eine relative Verschiebung der Position eines nachgezogenen Objekts in Relation zu der Position des vorgelegten Objekts evaluiert wird.

11. Verfahren nach einem der vorhergehenden Ansprüche, wobei die Linien des vorgelegten Objekts unter Verwendung von mindestens zwei Bewegungsrichtungen nachgezogen werden.

12. Verfahren nach einem der vorhergehenden Ansprüche, wobei die Reihenfolge der nachgezogenen Linien evaluiert wird.

**13.** Verfahren nach einem der vorhergehenden Ansprüche, wobei die Linienbreite und/oder der durch den Benutzer beim Nachziehen eines Objekts aufgebrachte Druck evaluiert werden.

**14.** Verfahren nach einem der vorhergehenden Ansprüche, wobei die Innenseite des vorgelegten linienförmigen Objekts durch den Benutzer gefärbt werden muss und wobei die Konformität des Färbens mit den Randlinien des vorgelegten Objekts evaluiert wird.

**15.** Verfahren nach einem der vorhergehenden Ansprüche, wobei ein mindestens teilsweise verborgenes oder maskiertes linienförmiges Objekt vorgelegt wird.

**16.** Verfahren nach Anspruch 15, wobei mindestens zwei stützende Punkte eines vorgelegten Objekts vorgelegt werden.

**17.** Verfahren nach einem der vorhergehenden Ansprüche, wobei ein Objekt nur vorübergehend vorgelegt wird.

**18.** Computerimplementiertes System zum Testen und/oder Trainieren visueller, kognitiver und/oder Auge-Hand-Koordinationsfunktionen oder -fähigkeiten eines Benutzers, wobei das System ein berührungsempfindliches Display umfasst, **gekennzeichnet durch** Verarbeitungsmittel zum Ausführen des Verfahrens nach einem der vorhergehenden Ansprüche.

**19.** System nach Anspruch 18 umfassend Verarbeitungsmittel zum Vorlegen mindestens eines linienförmigen Objekts (100) auf dem berührungsempfindlichen Display zum Detektieren von Informationen über eine oder mehrere Linien des durch den Benutzer nachgezogenen (105) vorgelegten Objekts (100) unter Verwendung des berührungsempfindlichen Displays und zum Evaluieren der detektierten Informationen zum Bestimmen einer Charakteristik, die Abweichungen und/oder eine Konformität (120) zwischen dem vorgelegten mindestens einen linienförmigen Objekt (100) und der/ den nachgezogenen Linie (n) (105) betrifft.

**Revendications**

**1.** Procédé informatisé pour tester et/ou entraîner les fonctions ou les compétences visuelles, cognitives et/ou de coordination oeil-main d'un utilisateur, à l'aide d'un ordinateur ou d'un dispositif de traitement des données ayant un écran tactile, dans lequel au moins un objet en forme de ligne (100) est présenté sur l'écran tactile, dans lequel l'utilisateur doit retracer la ou les ligne (s) de l'objet (100) présenté et dans lequel la ou les ligne (s) (105) retracée (s) est/sont évaluée(s) afin de déterminer une caractéristique relative aux déviations et/ou à la conformité (120) entre l'au moins un objet en forme de ligne (100) présenté et la ou les ligne(s) (105) retracée(s), dans lequel la caractéristique est déterminée sur la base de l'évaluation du temps nécessaire pour retracer la ou les ligne (s) de l'objet présenté ;
**caractérisé en ce que** la caractéristique est en outre déterminée sur la base des étapes d'évaluation suivantes :

évaluation d'une distance de pixel moyenne pour des pixels dessinés à une certaine distance du pixel le plus proche présenté à l'origine ;
évaluation d'une variance moyenne donnant des informations sur la régularité avec laquelle le dessin est réalisé ;
évaluation d'une longueur de courbe par rapport à une longueur de courbe d'origine ;
évaluation d'une distance de pixel inverse moyenne donnant des informations sur l'éloignement d'un pixel d'origine par rapport au pixel dessiné le plus proche.

**2.** Procédé selon la revendication 1, dans lequel la caractéristique déterminée est utilisée pour déterminer les fonctions visuelles, cognitives et/ou de coordination oeil-main de l'utilisateur ou l'état de santé de l'utilisateur.

**3.** Procédé selon la revendication 1 ou 2, dans lequel la caractéristique est un nombre scalaire ou un nombre multidimensionel.

**4.** Procédé selon l'une quelconque des revendications précédentes, dans lequel la caractéristique est déterminée par une somme ou une intégration linéaire ou non linéaire de valeurs de déviation uniques.

**5.** Procédé selon l'une quelconque des revendications précédentes, dans lequel le temps nécessaire pour retracer la ou les ligne(s) de l'objet présenté est évaluée.

**6.** Procédé selon l'une quelconque des revendications précédentes, dans lequel le calcul de la distance de pixel moyenne (apd) est réalisé sur la base de l'équation (1) suivants :

$$apd = \frac{\sum_{i=1}^{n} distance(P(i)_{dessin}, P(i)_{original})}{n} \; ; \qquad (1)$$

dans laquelle le calcul de la variance moyenne (var) est réalisé sur la base de l'équation (2) suivants :

$$var = \frac{\sum_{i=1}^{n}(distance(P(i)_{dessin}, P(i)_{original}) - apd)^2}{n} \; ; \qquad (2)$$

dans laquelle le calcul de la longueur de courbe (cl) et d'une différence de longueur de courbe (cld) correspondante est réalisé sur la base des deux équations (3) suivantes :

$$cl = \sum_{i=1}^{m-1} \sqrt{(DP(x)_{i+1} - DP(x)_i)^2 + (DP(y)_{i+1} - DP(y)_i)^2} \qquad (3)$$

$$cld = |cl_{dessin} - cl_{original}|;$$

dans laquelle le calcul de la distance de pixel inverse moyenne (ipd) est réalisé sur la base de l'équation (4) suivante :

$$ipd = \frac{\sum_{i=1}^{n} distance(P(i)_{original}, P(i)_{dessin})}{n} \; ; \qquad (4)$$

dans laquelle le temps nécessaire pour retracer la ou les ligne (s) de l'objet présenté est mesuré en secondes.

**7.** Procédé selon la revendication 6, dans lequel les quotients modulaires suivants sont calculés :

$$q_{apd} = \min_{100}(\max_{0} 100 \frac{apd_{pire} - apd}{apd_{pire} - apd_{meilleure}})$$

$$q_{var} = \min_{100}(\max_{0} 100 \frac{var_{pire} - var}{var_{pire} - var_{meilleure}})$$

$$q_{cld} = \min_{100}(\max_{0} 100 \frac{cld_{pire} - cld}{cld_{pire} - cld_{meilleure}})$$

$$q_{ipd} = \min_{100}(\max_{0} 100 \frac{ipd_{pire} - ipd}{ipd_{pire} - ipd_{meilleure}})$$

$$q_{time} = \min_{100}(\max_{0} 100 \frac{temps_{pire} - temps}{temps_{pire} - temps_{meilleure}})$$

**8.** Procédé selon la revendication 7, dans lequel, sur la base des quotients modulaires, un score principal est calculé sous la forme d'un score moyen (score$_{avg}$) ou d'un score multiplicatif (score$_{mult}$), comme suit :

$$score_{avg} = \frac{q_{apd} + q_{var} + q_{cld} + q_{ipd} + q_{temps}}{5}$$

ou

$$score_{mult} = 100 \left( \frac{q_{apd}}{100} \frac{q_{var}}{100} \frac{q_{cld}}{100} \frac{q_{ipd}}{100} \frac{q_{temps}}{100} \right).$$

**9.** Procédé selon l'une quelconque des revendications 5 à 8, dans lequel le temps écoulé entre le dessin de deux lignes de l'objet présenté est évaluée, ledit temps écoulé étant notamment comparé aux données correspondantes prévues de patients d'essai ayant une certaine maladie.

**10.** Procédé selon l'une quelconque des revendications précédentes, dans lequel un déplacement relatif de la position d'un objet retracé par rapport à la position de l'objet présenté est évaluée.

**11.** Procédé selon l'une quelconque des revendications précédentes, dans lequel les lignes de l'objet présenté sont retracées à l'aide d'au moins deux directions de déplacement.

**12.** Procédé selon l'une quelconque des revendications précédentes, dans lequel l'ordre des lignes retracées est évalué.

**13.** Procédé selon l'une quelconque des revendications précédentes, dans lequel la largueur de ligne et/ou de la pression appliquée par l'utilisateur lorsqu'un objet est retracé sont évaluées.

**14.** Procédé selon l'une quelconque des revendications précédentes, dans lequel l'intérieur d'un objet en forme de ligne présenté doit être colorié par l'utilisateur et dans lequel la conformité de la couleur avec les lignes de bordure de l'objet présenté est évaluée.

**15.** Procédé selon l'une quelconque des revendications précédentes, dans lequel un objet en forme de ligne au moins en partie caché ou masqué est présentée.

**16.** Procédé selon la revendication 15, dans lequel au moins deux points d'appui d'un objet présenté sont présentés.

**17.** Procédé selon l'une quelconque des revendications précédentes, dans lequel un objet est présenté uniquement temporairement.

**18.** Système informatisé pour tester et/ou entraîner des fonctions ou des compétences visuelles, cognitives et/ou de coordination oeil-main d'un utilisateur, dans lequel le système comprend un écran tactile, **caractérisé par** la présence de moyens de traitement pour exécuter le procédé selon l'une quelconque des revendications précédentes.

**19.** Système selon la revendication 18, comprenant des moyens de traitement pour présenter au moins un objet en forme de ligne (100) sur l'écran tactile, pour détecter des informations sur la ou les ligne (s) de l'objet (100) présenté retracé (105) par l'utilisateur, à l'aide de l'écran tactile, et pour évaluer les informations détectées afin de déterminer une caractéristique relative aux déviations et/ou à la conformité (120) entre l'au moins un objet en forme de ligne (100) présenté et la ou les ligne (s) (105) retracée (s).

FIG. 1 A-C

A.

B.

FIG. 2 A-B

FIG. 4 A-B

FIG. 3 A-B

A.

B.

FIG. 5 A-B

C.

D.

**FIG. 5 C-D**

A.

B.

FIG. 6 A-B

FIG. 7A

FIG. 7B

A.

B.

C.

# FIG. 8 A-C

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008128192 A1
- WO 2013111746 A1
- US 2012035498 A1
- WO 2011063248 A1
- US 5397865 A
- WO 9832107 A1
- US 2014045148 A1